# EUROPEAN PATENT APPLICATION

(11) **EP 2 062 877 A1**
(43) Date of publication of application: **27.05.2009**
(21) Application number: 08253647.5
(22) Date of filing: 07.11.2008
(51) Int. Cl.: C07C 263/20, C08G 18/10, C08G 18/42, C08G 18/48, C08G 18/76, C09J 175/04

(54) **Purification of isocyanate functional polymers**

(30) Priority: 20.11.2007 US 989209 P; 30.10.2008 US 261505
(71) Applicant: Tyco Healthcare Group LP, North Haven, CT 06473 (US)
(72) Inventor: Calabrese, Allison Foss, Hamden, CT 06517 (US); Skalla, Walter, Old Lyme, CT 06371 (US); Belcheva, Nadya, Hamden, CT 06518 (US)
(74) Representative: Soames, Candida Jane

(57) **Abstract**

Methods are provided for isolating and purifying components useful, either alone or in combination with other components, as adhesives or sealants for medical/surgical applications.

## Description

### CROSS-REFERENCE TO RELATED APPLICATIONS

This application claims the benefit of and priority to U.S. Provisional Patent Application No. 60/989,209, filed November 20, 2007, the entire disclosure of which is incorporated by reference herein.

### TECHNICAL FIELD

The present disclosure relates to processes which may be utilized in obtaining and/or purifying isocyanate-functional polymers. These isocyanate-functional polymers may be utilized, in embodiments, in the formation of surgical adhesives or sealants.

### RELATED ART

There has developed an increased interest in replacing or augmenting sutures with adhesive bonds. The reasons for this increased interest include: (1) the potential speed with which repair might be accomplished; (2) the ability of a bonding substance to effect complete closure, thus preventing seepage of fluids; and (3) the possibility of forming a bond without excessive deformation of tissue.

Studies in this area, however, have revealed that in order for surgical adhesives to be accepted by surgeons, they must possess a number of properties. They must exhibit high initial tack and an ability to bond rapidly to living tissue; the strength of the bond should be sufficiently high to cause tissue failure before bond failure; the adhesive should form a bridge, typically a permeable flexible bridge; and the adhesive bridge and/or its metabolic products should not cause local histotoxic or carcinogenic effects.

Methods for producing these adhesives and/or sealants, as well as the components thereof, are within the purview of those skilled in the art. It may be desirable in the synthesis of these components to obtain the desired component with little residual starting materials, solvents, by-products, and the like, so that any resulting adhesive or sealant possesses little, if any, residual starting materials, solvents, by-products, and the like.

### SUMMARY

Methods are provided for isolating and purifying components useful, either alone or in combination with other components, as adhesives or sealants for medical/surgical applications.

In embodiments, methods of the present disclosure include contacting a polymer possessing isocyanate groups with a solvent to form a solution, adding a metal oxide selected from the group consisting of aluminum oxide, titanium oxide, iron oxide, magnesium oxide, zinc oxide, silica, and combinations thereof to the solution, and isolating the polymer possessing isocyanate groups from the solution.

In other embodiments, methods of the present disclosure may include forming a solution by contacting a solvent with at least one poly(ether-ester) macromer possessing isocyanate groups of the formula:

OCN-X-HNCOO-(R-A)ₙ-ROOCNH-X-NCO (II)

wherein X is a group derived from toluene, xylene, isophorone, diphenylmethane, diphenyldimethylmethane, dibenzyl diisocyanate, oxybis(phenylisocyanate), tetramethylxylylene, hexamethylene, tetramethylene, lysine, ethylated lysine, and combinations thereof, A is a group derived from an aliphatic diacid, R is a group derived from a polyethylene glycol, and n is 1 to 10. A metal oxide may then be added to the solution. Suitable metal oxides include acidic aluminum oxide, basic aluminum oxide, neutral aluminum oxide, and combinations thereof in an amount from about 2% w/v to about 20% w/v. At least one poly(ether-ester) macromer possessing isocyanate groups may then be isolated from the solution.

### DETAILED DESCRIPTION

The present disclosure relates to processes for producing and obtaining components which, in turn, may be utilized in the synthesis of bioabsorbable compositions which may be used as tissue adhesives or sealants. As used herein, a "composition" of the present disclosure includes the above components, either by themselves or with optional additives and/or additional components.

In embodiments, the processes of the present disclosure may be useful in obtaining and/or purifying isocyanate-functional polymers. Any isocyanate-functional polymers may be obtained from any media utilized in their synthesis utilizing the methods of the present disclosure. In some embodiments, the isocyanate-functional polymers may include isocyanate-functional poly(ether-ester) macromers. These macromers may include an aliphatic diacid linking two dihydroxy components (sometimes referred to herein as a "poly(ether-ester) macromer"). Up to ten repeats of the poly(ether-ester) macromer may be present.

Suitable aliphatic diacids which may be utilized in forming the poly(ether-ester) macromer include, for example, aliphatic diacids having from about 2 to about 10 carbon atoms. Suitable diacids include, but are not limited to, sebacic acid, azelaic acid, suberic acid, pimelic acid, adipic acid, glutaric acid, succinic acid, malonic acid, oxalic acid, and combinations thereof.

Suitable dihydroxy components which may be utilized include, for example, polyols including polyalkylene oxides, polyvinyl alcohols, and the like. In some embodiments, the dihydroxy components can be a polyalkylene oxide such as polyethylene oxide ("PEO"), polypropylene oxide ("PPO"), block or random copolymers of polyethylene oxide (PEO) and polypropylene oxide (PPO), and combinations thereof.

In one embodiment, a polyethylene glycol ("PEG") may be utilized as the dihydroxy component. It may be desirable to utilize a PEG with a molecular weight of from about 200 g/mol to about 10000 g/mol, in embodiments from about 400 g/mol to about 900 g/mol. Suitable PEGs include those commercially available from a variety of sources under the designations PEG 200, PEG 400, PEG 600 and PEG 900.

Any method may be used to form the poly(ether-ester) macromer. In some embodiments, the poly(ether-ester) macromer may be formed by combining adipoyl chloride with a PEG such as PEG 600 and pyridine in a suitable solvent, such as tetrahydrofuran (THF). The solution may be held at a suitable temperature, from about -70° C to about 25° C, for a period of time of from about 4 hours to about 18 hours, after which the reaction mixture is filtered to remove the precipitated pyridine hydrochloride by-product and the resulting poly(ether-ester) macromer, here a PEG/adipate component, may be obtained from the solution by the addition of ether or petroleum ether, and collected by suitable means which can include filtration. Other methods suitable for making the present components are within the purview of those skilled in the art.

In embodiments, the resulting poly(ether-ester) macromer may be of the following formula:

HO-(R-A)ₙ-R-OH (I)

wherein A is a group derived from an aliphatic diacid; R can be the same or different at each occurrence and may include a group derived from a dihydroxy component; and n may be from about 1 to about 10. In some embodiments, the A group can be derived from adipic acid, and R can be derived from a polyethylene glycol having a molecular weight of from about 200 g/mol to about 1000 g/mol, in embodiments from about 400 g/mol to about 800 g/mol, in embodiments about 600 g/mol. The molecular weight and viscosity of these components may depend on a number of factors such as the particular diacid used, the particular dihydroxy component used, and the number of repeat units present. Generally, the viscosity of these components may be from about 300 to about 10,000 cP at 25°C and a shear rate of 20.25 sec⁻¹.

These poly(ether-ester) components may be useful for a number of applications. For example, they may be used to produce compositions capable of cross-linking to form a gel matrix that serves as an excellent tissue adhesive or sealant. For adhesive or sealant applications, it may be desirable to endcap the above poly(ether-ester) macromer to provide a reactive end group. Suitable reactive end groups include amine reactive end groups, for example, isocyanate groups, isothiocyanates, diimidazoles, imidoesters, hydroxysuccinimide esters, and aldehydes.

In embodiments, the poly(ether-ester) component may be endcapped with isocyanate groups. Methods for endcapping the poly(ether-ester) macromer to provide a reactive end group are within the purview of those skilled in the art.

For example, the poly(ether-ester) macromer may be reacted with an aliphatic or aromatic diisocyanate to produce a diisocyanate-functional component. Suitable isocyanates for endcapping the poly(ether-ester) macromer include aromatic, aliphatic and alicyclic isocyanates. Examples include, but are not limited to, aromatic diisocyanates such as 2,4-toluene diisocyanate, 2,6-toluene diisocyanate, 2,2'-diphenylmethane diisocyanate, 2,4'-diphenylmethane diisocyanate, 4,4'-diphenylmethane diisocyanate, diphenyldimethylmethane diisocyanate, dibenzyl diisocyanate, naphthylene diisocyanate, phenylene diisocyanate, xylylene diisocyanate, 4,4'-oxybis(phenyl isocyanate), 4,4'-methylenebis(phenyl isocyanate), or tetramethylxylylene diisocyanate; aliphatic diisocyanates such as tetramethylene diisocyanate, hexamethylene diisocyanate, dimethyl diisocyanate, lysine diisocyanate, 2-methylpentane-1,5-diisocyanate, 3-methylpentane-1,5-diisocyanate or 2,2,4-trimethylhexamethylene diisocyanate; and alicyclic diisocyanates such as isophorone diisocyanate, cyclohexane diisocyanate, hydrogenated xylylene diisocyanate, hydrogenated diphenylmethane diisocyanate, hydrogenated trimethylxylylene diisocyanate, 2,4,6-trimethyl 1,3-phenylene diisocyanate, or commercially available materials including those sold under the DESMODURS^{®} name from Bayer Material Science.

Methods for endcapping the poly(ether-ester) macromer with a diisocyanate are within the purview of those skilled in the art. For example, the poly(ether-ester) macromer may be combined with a suitable diisocyanate at a molar ratio of poly(ether-ester) macromer to diisocyanate of from about 1:2 to about 1:6, in embodiments from about 1:3 to about 1:5, in other embodiments about 1:4, and heated to a suitable temperature of from about 55° C to about 75° C, in embodiments from about 60° C to about 70° C, in other embodiments about 65° C. It may be desirable to agitate the components utilizing means within the purview of those skilled in the art, including stirring, mixing, blending, sonication, combinations thereof, and the like. In some embodiments, the endcapping reaction may occur under an inert atmosphere, for example, under nitrogen gas. Catalysts, including alkoxides, pyridine components, combinations thereof, and the like, may be utilized in some embodiments.

It may be desirable, in embodiments, to utilize an excess of the diisocyanate in carrying out the reaction. The use of an excess of diisocyanate may suppress the polymerization reaction, thereby permitting one to tailor the resulting molecular weight of the resulting isocyanate functionalized poly(ether-ester) macromer.

The resulting isocyanate-functional poly(ether-ester) macromers may be of the following formula:

OCN-X-HNCOO-(R-A)ₙ-R-OOCNH-X-NCO (II)

wherein X is an aliphatic or aromatic group derived from the diisocyanate described above; A is a group derived from an aliphatic diacid; R can be the same or different at each occurrence and is a group derived from a dihydroxy component; and n is a number from about 1 to about 10. In some embodiments, X may derived from toluene, hexamethylene, 4,4'-methylenebis(phenyl), tetramethylene, lysine, ethylated lysine isophorone, xylene, diphenylmethane, diphenyldimethylmethane, dibenzyl diisocyanate, oxybis(phenylisocyanate), tetramethylxylylene, or optionally mixtures thereof or combinations thereof.

It should be understood that more than one different poly(ether-ester) macromer can be endcapped in a single reaction. For example, poly(ether-ester) macromers of the above-mentioned formula wherein n is 3 can be prepared and combined with poly(ether-ester) macromers of the above-mentioned formula wherein n is 5. The mixture of poly(ether-ester) macromers can then be endcapped to provide a reactive group in a single reaction. The resulting product will be a mixture of diisocyanate-functional components of the formula shown above.

The NCO content of the diisocyanate-functional component can vary from about 3% to about 6%, in embodiments from about 3.5% to about 5%. The viscosity of these diisocyanate-functional components will depend on a number of factors such as the particular diisocyanate used, the particular diacid used, the particular dihydroxy component used, and the number of repeat units present. Generally, the viscosity of these components may be from about 1,500 to about 50,000 centipoise ("cP"), in embodiments from about 3000 to about 20000 cP.

After completion of the above condensation reaction whereby the poly(ether-ester) macromer has been endcapped with the diisocyanate, the crude isocyanate-functional poly(ether-ester) macromers may be dissolved in a solvent. Suitable solvents are within the purview of those skilled in the art and include, for example, tetrahydrofuran, dioxane, dimethylformamide, dichloromethane, dimethylacetamide, gamma-butyrolactone, N-methylpyrollidone, ketones such as methyl ethyl ketone, cyclohexanone, diethylene glycol monoethyl ether acetate, diethylene glycol monobutyl ether acetate, diethylene glycol monomethyl ether, diethylene glycol monoethyl ether, diethylene glycol monobutyl ether, diethylene glycol monoisobutyl ether, diisobutyl ketone, diacetone alcohol, ethyl amyl ketone, ethyl lactate, ethyl acetate, isopropyl acetate, butyl acetate, isopropanol, butanol, acetone, combinations thereof, and the like. In embodiments, an organic solvent such as tetrahydrofuran, dioxane, dimethylformamide, dichloromethane, ethyl acetate, combinations thereof, and the like, may be utilized.

The amounts of solvent used will depend on a number of factors including the particular reactive component employed and the intended end use of the composition. Generally, the amount of solvent will be from about 95% to about 50%, in embodiments from about 90% to about 70%.

Once the solution of isocyanate-functional poly(ether-ester) macromers in solvent has been prepared, a metal oxide may be added thereto. Suitable metal oxides include, but are not limited to, aluminum oxide, titanium oxide, iron oxide, magnesium oxide, zinc oxide, silica, combinations thereof, and the like. In embodiments it may be desirable that the metal oxide be micronized, i.e., have a particle size of from about 0.05 mm to about 0.2mm, in embodiments from about 0.1 mm to about 0.15 mm.

In embodiments, an aluminum oxide may be added to the solution including isocyanate-functional poly(ether-ester) macromers. The aluminum oxide may be acidic, basic, neutral, or a combination thereof.

The metal oxide may be added to the solution of isocyanate-functional poly(ether-ester) macromers in an amount from about 2% w/v to about 20% w/v, in embodiments from about 5% w/v to about 15% w/v, in embodiments about 10%w/v. The resulting composition may then be physically agitated by mixing, blending, stirring, sonication, shaking, combinations thereof, and the like, for a suitable period of time of from about 20 minutes to about 4 hours, in embodiments from about 1 hour to about 3 hours, in embodiments about 2 hours. In embodiments, mixing at a speed of from about 200 revolutions per minute (rpm) to about 1000 rpm may be utilized, in other embodiments from about 500 rpm to about 700 rpm. Mixing may occur at any suitable temperature, in embodiments, from about 20º C to about 24º C. In embodiments, the metal oxide may function as a scavenger to assist in the elimination of any excess free un-reacted diisocyanates, any low molecular weight impurities, such as residual polyethylene glycols, traces of catalysts, such as alkoxide and/or pyridine catalysts, combinations thereof, and the like. Thus, the addition of the metal oxide may be useful in obtaining and/or purifying isocyanate-functional polymers as described above.

The isocyanate-functional poly(ether-ester) macromers may then be isolated by any means within the purview of those skilled in the art including filtration, solvent evaporation, combinations thereof, and the like. In embodiments, the isocyanate-functional poly(ether-ester) macromers may be isolated by filtration of the metal oxide and any free un-reacted diisocyanates, any low molecular weight impurities, such as residual polyethylene glycols, traces of alkoxide catalysts, combinations thereof, and the like, and evaporation of the solvent. The final purified product thus obtained may then be subjected to a drying step, either by subjecting the product to a vacuum, or by heating the product to a temperature of from about 30º C to about 70º C, in embodiments from about 40º C to about 50º C. By utilizing the metal oxide described above, precipitation and washing steps with additional solvents, which may be the source of additional contaminants or unwanted by-products, may thus be avoided.

The functionalized components described above can be used alone or can be formulated into compositions. The concentrations of the components utilized to form the compositions will vary depending upon a number of factors, including the types and molecular weights of the particular components used and the desired end use application of the biocompatible composition, e.g., an adhesive or sealant. Generally, the composition may contain from about 0.5% to about 100 % of the previously described functionalized macromer component, in embodiments from about 0.5% to about 100 % of the previously described functionalized macromer component.

If the viscosity of the components of the present disclosure is deemed too high for a particular application, solutions or emulsions may be formulated that include a solvent in addition to the compositions. Suitable solvents which may be utilized include, for example, polar solvents such as water, ethanol, triethylene glycol, glymes (such as diglyme, triglyme, tetraglyme, and the like), polyethylene glycols, methoxy-polyethylene glycols, dimethylformamide, dimethylacetamide, gamma-butyrolactone, N-methylpyrollidone, ketones such as methyl ethyl ketone, cyclohexanone, diethylene glycol monoethyl ether acetate, diethylene glycol monobutyl ether acetate, diethylene glycol monomethyl ether, diethylene glycol monoethyl ether, diethylene glycol monobutyl ether, diethylene glycol monoisobutyl ether, diisobutyl ketone, diacetone alcohol, ethyl amyl ketone, ethyl lactate, and the like, and mixtures thereof. In other embodiments, solvents such as tetrahydrofuran, ethyl acetate, isopropyl acetate, butyl acetate, isopropanol, butanol, acetone, mixtures thereof, and the like, may be utilized.

The amount of solvent used will depend on a number of factors including the particular reactive component employed and the intended end use of the composition. Generally, the solvent may be from about 1 to about 50 weight percent of the entire composition, in embodiments from about 10 to about 30 weight percent of the entire composition. The use of one or more solvents can produce an emulsion having a viscosity of from about 100 cP to about 1500 cP, in embodiments from about 500 cP to about 1000 cP. Such emulsions can advantageously be sprayed using any suitable spraying device.

Where the functionalized macromer component includes isocyanate functionality and the solvent contains hydroxyl groups, the solvent may be advantageously mixed with the functionalized macromer component immediately prior to use to avoid undesired pre-gelling. In other embodiments, as noted above, the component possessing isocyanate functionality, i.e. isocyanate-functional poly(ether-ester) macromers, may be applied to tissue whereby endogenous water causes cross-linking of the isocyanate-functional poly(ether-ester) macromers thereby forming an adhesive or sealant in situ.

Water may also be added to the composition to decrease cure time. When added, water should be introduced at or near the time of use of the composition to avoid unwanted or pre-mature crosslinking. Generally, the amount of water may be from about 1 to about 50 weight percent based on the entire composition.

Compositions in accordance with this disclosure may optionally include one or more catalysts. The addition of a catalyst can decrease the cure time of the compositions of the present disclosure. Catalysts which may be utilized include Lewis acids, tertiary amine catalysts, quaternary amine catalysts, and the like.

Suitable tertiary amine catalysts which may be added include, but are not limited to, triethylenediamine, N-methylmorpholine, pentamethyl diethylenetriamine, dimethylcyclohexylamine, tetramethylethylenediamine, 1-methyl-4-dimethylaminoethyl-piperazine, 3-methoxy-N-dimethyl-propylamine, N-ethylmorpholine, diethylethanolamine, N-cocomorpholine, N,N-dimethyl-N',N'-dimethylisopropyl-propylene diamine, N,N-diethyl-3-diethyl aminopropylamine and dimethyl-benzyl amine.

Suitable quaternary amine catalysts include, for example, lower alkyl ammonium halides and their derivatives such as hydroxy, chlorhydrin and epoxy substituted lower alkyl trimethylammonium halides such as substituted propyltrimethylammonium chlorides. Quaternary amines which may be utilized include dihydroxypropyltrimethylammonium chloride, chlorohydroxypropyltrimethylammonium chloride, and epoxypropyltrimethylammonium chloride. Specific examples of the above components include 3-chloro-2-hydroxypropyl trimethyl ammonium chloride, 2,3-epoxypropyl trimethyl ammonium chloride, 3-chloro-2-hydroxypropyl trimethyl ammonium chloride, and 2,3-dihydroxypropyltrimethyl ammonium chloride.

In other embodiments, catalysts for use in the cross-linking reaction include 1,4-diazobicyclo [2.2.2] octane, stannous octoate, and the like.

The amount of catalyst employed can range from about 0.5 grams to about 50 grams per kilogram of the component being cross-linked. In one embodiment, the amount of catalyst ranges from about 0.5 grams to about 10 grams per kilogram of the component being cross-linked.

A variety of optional ingredients may also be added to the compositions of the present disclosure, including but not limited to surfactants, antimicrobial agents, colorants, preservatives, imaging agents e.g., iodine or barium sulfate, or fluorine, or medicinal agents. In some embodiments, the present compositions may optionally contain one or more bioactive agents. The term "bioactive agent", as used herein, is used in its broadest sense and includes any substance or mixture of substances that have clinical use. Consequently, bioactive agents may or may not have pharmacological activity per se, e.g., a dye. Alternatively, a bioactive agent could be any agent which provides a therapeutic or prophylactic effect, a component that affects or participates in tissue growth, cell growth, cell differentiation, a component that may be able to invoke a biological action such as an immune response, or could play any other role in one or more biological processes.

Examples of classes of bioactive agents which may be utilized in accordance with the present disclosure include antimicrobials, analgesics, antipyretics, anesthetics, antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, and enzymes. It is also intended that combinations of bioactive agents may be used.

Suitable antimicrobial agents which may be included as a bioactive agent in the present compositions include triclosan, also known as 2,4,4'-trichloro-2'-hydroxydiphenyl ether, chlorhexidine and its salts, including chlorhexidine acetate, chlorhexidine gluconate, chlorhexidine hydrochloride, and chlorhexidine sulfate, silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol 9, fusidic acid, cephalosporins, and combinations thereof. In addition, antimicrobial proteins and peptides such as bovine or rh-lactoferrin and lactoferricin B may be included as a bioactive agent in the present compositions.

Other bioactive agents which may be included as a bioactive agent in the present compositions include: local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents (e.g. oxybutynin); antitussives; bronchodilators; cardiovascular agents such as coronary vasodilators and nitroglycerin; alkaloids; analgesics; narcotics such as codeine, dihydrocodeinone, meperidine, morphine and the like; non-narcotics such as salicylates, aspirin, acetaminophen, d-propoxyphene and the like; opioid receptor antagonists, such as naltrexone and naloxone; anti-cancer agents; anticonvulsants; anti-emetics; antihistamines; anti-inflammatory agents such as hormonal agents, hydrocortisone, prednisolone, prednisone, non-hormonal agents, allopurinol, indomethacin, phenylbutazone and the like; prostaglandins and cytotoxic drugs; estrogens; antibacterials; antibiotics; anti-fungals; anti-virals; anticoagulants; anticonvulsants; antidepressants; antihistamines; and immunological agents.

Other examples of suitable bioactive agents which may be included in the present compositions include viruses and cells, peptides, polypeptides and proteins, analogs, muteins, and active fragments thereof, such as immunoglobulins, antibodies, cytokines (e.g. lymphokines, monokines, chemokines), blood clotting factors, hemopoietic factors, interleukins (IL-2, IL-3, IL-4, IL-6), interferons (β-IFN, (α-IFN and γ-IFN), erythropoietin, nucleases, tumor necrosis factor, colony stimulating factors (e.g., GCSF, GM-CSF, MCSF), insulin, anti-tumor agents and tumor suppressors, blood proteins, gonadotropins (e.g., FSH, LH, CG, etc.), hormones and hormone analogs (e.g., growth hormone), vaccines (e.g., tumoral, bacterial and viral antigens); somatostatin; antigens; blood coagulation factors; growth factors (e.g., nerve growth factor, insulin-like growth factor); protein inhibitors, protein antagonists, and protein agonists; nucleic acids, such as antisense molecules, DNA and RNA; oligonucleotides; and ribozymes.

Naturally occurring polymers, including proteins such as collagen and derivatives of various naturally occurring polysaccharides such as glycosaminoglycans, can optionally be incorporated into the compositions the bioactive agent of the present disclosure.

A single bioactive agent may be utilized to form the present compositions or, in alternate embodiments, any combination of bioactive agents may be utilized to form the present compositions.

Due to the presence of the functionalized macromer components described above, the present compositions cross-link to form a gel matrix that serves as an excellent tissue adhesive or sealant upon administration to tissue. Normally, the cross-linking reaction may be conducted at temperatures of from about 20°C to about 40° C, in embodiments from about 25°C to about 37° C, for a period of time of from about fifteen seconds to about 20 minutes, in embodiments from about 30 seconds to about 10 minutes. The exact reaction conditions for achieving cross-linking of the compositions of the present disclosure depend upon a variety of factors, including the functionality of the component, the degree of endcapping, the degree of functionalization, the presence of a catalyst, the particular solvent, if any, and the like.

Where the composition of the present disclosure is intended for delivery of a drug or protein, the amounts of the functionalized components of the present disclosure can be adjusted to promote the initial retention of the drug or polymer in the bioabsorbable composition and its subsequent release. Methods and means for making such adjustments will be readily apparent to those skilled in the art.

The compositions of the present disclosure can be used for a number of different human and animal medical applications including, but not limited to, wound closure (including surgical incisions and other wounds). Adhesives may be used to bind tissue together either as a replacement of, or as a supplement to, sutures, staples, clamps, tapes, bandages, and the like. Use of the present compositions can eliminate or substantially reduce the number of sutures normally required during current practices, and eliminate the subsequent need for removal of staples and certain types of sutures. The compositions described herein can thus be particularly suitable for use with delicate tissues where sutures, clamps or other conventional tissue closure mechanisms may cause further tissue damage. For example, the compositions of the present disclosure may be used to seal or adhere delicate tissue together, such as lung tissue, in place of conventional tools that may cause mechanical stress. The present compositions can also be used to seal air and/or fluid leaks in tissue as well as to prevent post-surgical adhesions and to fill voids and/or defects in tissue.

To effectuate the joining of two tissue edges, the two edges may be approximated, and a composition of the present disclosure may be applied to the two approximated edges. The composition crosslinks rapidly, generally taking less than one minute. Compositions of the present disclosure can thus be applied to the wound and allowed to set, thereby closing the wound.

While certain distinctions may be drawn between the usage of the terms "flesh" and "tissue" within the scientific community, the terms are used interchangeably herein as referring to a general substrate upon which those skilled in the art would understand the present bioabsorbable composition to be utilized within the medical field for the treatment of patients. As used herein, "tissue" may include, but is not limited to, skin, bone, neuron, axon, cartilage, blood vessel, cornea, muscle, fascia, brain, prostate, breast, endometrium, lung, pancreas, small intestine, blood, liver, testes, ovaries, cervix, colon, stomach, esophagus, spleen, lymph node, bone marrow, kidney, peripheral blood, embryonic and/or ascite tissue.

The compositions described herein can also be used as sealants. When used as a sealant, a component of the present disclosure can be used in surgery to form a bioabsorbable composition to prevent or inhibit bleeding or fluid leakage both during and after a surgical procedure. It can also be applied to prevent air leaks associated with pulmonary surgery. Compositions and/or components herein may be applied directly to the desired area in at least an amount sufficient to seal off any defect in the tissue and seal off any fluid or air movement. The compositions may also be used to prevent or control blood or other fluid leaks at suture or staple lines.

The present compositions also can be used to attach skin grafts and position tissue flaps during reconstructive surgery. Alternatively, the present compositions can be used to close tissue flaps in periodontal surgery.

Application of the compositions of the present disclosure can be done by any conventional means. These include dripping, brushing, or other direct manipulation of the compositions on the tissue surface, or spraying of the compositions onto the surface. In open surgery, application by hand, forceps or the like is contemplated. In endoscopic surgery, the compositions can be delivered through the cannula of a trocar, and spread at the site by any device known in the art.

In other embodiments, especially where a composition of the present disclosure is to be utilized as a void filler or sealant to fill a defect in an animal's body, it may be advantageous to more precisely control the conditions and extent of cross-linking. For example, it may be desirable to partially cross-link the composition prior to use to fill a void in animal tissue. In such a case composition of the present disclosure can be applied to the void or defect and allowed to set, thereby filling the void or defect.

In another embodiment, the present disclosure is directed to a method for using components of the present disclosure to adhere a medical device to tissue. Suitable medical devices include implants. Other medical devices include, but are not limited to, pacemakers, stents, shunts and the like. Generally, for adhering a device to the surface of animal tissue, a composition of the present disclosure can be applied to the device, to the tissue surface or to both. The device and tissue surface are then brought into contact with the present composition therebetween. Once the composition crosslinks and sets, the device and tissue surface are effectively adhered to each other.

The present compositions can also be used to prevent post surgical adhesions. In such an application, a composition of the present disclosure is applied and cured to form a layer on surfaces of internal tissues in order to prevent the formation of adhesions at a surgical site during the healing process.

The resulting bioabsorbable composition has a number of advantageous properties. The bioabsorbable compositions of the present disclosure are safe, possess enhanced adherence to tissue, are biodegradable, have enhanced hemostatic potential, have low cost, and are easy to prepare and use. By varying the selection of the components utilized to form the bioabsorbable composition, the strength and elasticity of the bioabsorbable composition can be controlled, as can the gelation time.

The components herein rapidly form a compliant gel matrix as the bioabsorbable composition, which insures stationary positioning of tissue edges or implanted medical devices in the desired location and lowers overall required surgical/application time. The resulting bioabsorbable composition exhibits little or no swelling upon gel matrix formation, and therefore retains the positional integrity of the aligned tissue edges and/or location of a medical device. The bioabsorbable composition forms strong cohesive bonds. It exhibits excellent mechanical performance and strength, while retaining the necessary pliability to adhere living tissue. This strength and pliability allows a degree of movement of tissue without shifting the surgical tissue edge.

In order that those skilled in the art may be better able to practice the features of the present disclosure described herein, the following examples are provided to illustrate, but not limit, the features of the present disclosure.

### EXAMPLE 1

About 45 grams of PEG 600 (Sigma Aldrich, St. Louis, MO) were added to a clean, oven dried, and nitrogen cooled 1 liter, 3 neck flask. Then, about 300 ml to about 400 ml of tetrahydrofuran (THF) (JT Baker, Phillipsburg, NJ) was added to the flask. About 9.2 grams of adipoyl chloride (AdCl) (98 %, Sigma Aldrich, St. Louis, MO) dissolved in about 200 ml to about 250 ml of THF was then added to the flask. The contents were stirred for about 10 minutes. Then, about 8.7 grams of anhydrous pyridine (EMD Sciences, Gibbstown, NJ) was combined with about 60 ml of tetrahydrofuran (THF) (JT Baker, Phillipsburg, NJ) and added to the flask in three separate 20 ml additions.

The reaction was completed in about 2 to about 2.5 hours. The mixture was left overnight for about 16 to about 20 hours at room temperature of from about 20°C to about 24°C. The soluble fraction was measured in situ by infrared spectroscopy using a REACTIR™ 4000 Spectrometer (Mettler-Toledo AutoChem, Columbia, MD); the ReactlR probe was inserted into the flask; the background utilized was air. The spectrometer scan that was obtained confirmed the presence of PEG/AdCl at a molar ratio of about 3:2.

### EXAMPLE 2

Isocyanate endcapping of PEG adipate. A dry 1 liter three neck flask was outfitted with a mechanical stir assembly and dry condenser. The apparatus was placed in a dry room at about 2% relative humidity. About 40.65 grams of the PEG/adipate dissolved in THF from Example 1 was transferred to the flask. About 8.7 grams of toluene diisocyanate (TDI) (technical grade 80%, Sigma Aldrich, St. Louis, MO) was added to the flask so that the molar ratio of TDI to PEG/adipate was about 2:1. The resulting mixture was stirred while slowly refluxing the THF (from the PEG/adipate solution). After about 15 hours, the mixture had a slight yellow color. About 50 grams of neutral alumina (Al₂O₃) (from Sigma Aldrich) was then added and stirred for about 1 hour.

The solution was then filtered through a Millipore filtering system, using a filter possessing pores of about 0.45 µ in diameter; the filter was changed after approximately every 25 mL of solution had passed therethrough.

The filtered solution was concentrated on a ROTOVAPOR^{®} rotary evaporator (BÜCHI Labortechnik AG, Flawil, Switzerland) to remove most of the THF.

About 100 to about 200 mL of boiling petroleum ether was added and the ether was decanted after addition and mixing before placing the product under vacuum. The resulting mixture was allowed to dry under a vacuum at a temperature of about 65°C for a period of from about 90 to about 96 hours, producing an adhesive composition of the present disclosure.

Lap shear of the resulting composition was determined by as follows. Room temperature porcine stomach tissue was cut into pieces about 15 x 45 mm in size using a punch. The tissue was rinsed with saline and blotted to remove excess moisture. About 0.1 mL of the above adhesive composition was then applied to the end of one of the tissue pieces. The adhesive was spread around to cover an area of about 15 x 15 mm at the end of the tissue piece. Another tissue piece was placed on top of the area covered by the adhesive.

A 20 gram weight was placed on top of the adhered area for about 30 seconds. The weight was removed and the adhesive was let cure for about 4.5 minutes more, for a total of 5 minutes cure time. The end of one of the tissue pieces was placed into a grounding clamp, then the other end was placed into a second clamp mounted on a counter. A force meter was attached to the top clamp and the force required to pull the pieces apart was recorded. The adhesive material had a lap shear of about 0.366 kg.

NCO content was determined by titration on a TitroLine Alpha Autotitrator manufactured by Schott-Geräte. The average NCO content of the material was about 1.4%. The presence of the NCO endcapped PEG/adipate was confirmed by FTIR and NMR.

It will be understood that various modifications may be made to the embodiments disclosed herein. For example, the diisocyanate functionalized poly(ether-ester) macromer can be used to prepare polyurethanes and used for applications other than adhesives or sealants. Therefore the above description should not be construed as limiting, but merely as exemplifications of preferred embodiments. Those skilled in the art will envision other modifications within the scope and spirit of the claims appended hereto.

## Claims

1. A process comprising:
contacting a polymer possessing isocyanate groups with a solvent to form a solution;
adding to the solution a metal oxide selected from the group consisting of aluminum oxide, titanium oxide, iron oxide, magnesium oxide, zinc oxide, silica, and combinations thereof; and
isolating the polymer possessing isocyanate groups from the solution.

2. The process of claim 1, wherein the polymer possessing isocyanate groups is of the following formula:
OCN-X-HNCOO-(R-A)ₙ-ROOCNH-X-NCO (II)
wherein X is an aliphatic or aromatic group; A is a group derived from an aliphatic diacid; R can be the same or different at each occurrence and is a group derived from a dihydroxy component; and n is from about 1 to about 10.

3. The process of claim 2, wherein A is a group derived from adipic acid, and R is a group derived from a polyalkylene glycol.

4. The process of claim 3, wherein R is a group derived from a component selected from the group consisting of polyethylene glycol 400, polyethylene glycol 600 and polyethylene glycol 900.

5. The process of claim 2, wherein X is a group derived from toluene, xylene, isophorone, diphenylmethane, diphenyldimethylmethane, dibenzyl diisocyanate, oxybis(phenylisocyanate), tetramethylxylylene, hexamethylene, tetramethylene, lysine, ethylated lysine, and combinations thereof.

6. The process of claim 1, wherein the solvent is selected from the group consisting of tetrahydrofuran, dioxane, dimethylformamide, dichloromethane, dimethylacetamide, gamma-butyrolactone, N-methylpyrollidone, methyl ethyl ketone, cyclohexanone, diethylene glycol monoethyl ether acetate, diethylene glycol monobutyl ether acetate, diethylene glycol monomethyl ether, diethylene glycol monoethyl ether, diethylene glycol monobutyl ether, diethylene glycol monoisobutyl ether, diisobutyl ketone, diacetone alcohol, ethyl amyl ketone, ethyl lactate, ethyl acetate, isopropyl acetate, butyl acetate, isopropanol, butanol, acetone, and combinations thereof.

7. The process of claim 1, wherein the solvent comprises an organic solvent selected from the group consisting of tetrahydrofuran, dioxane, dimethylformamide, dichloromethane, ethyl acetate, and combinations thereof.

8. The process of claim 1, wherein the metal oxide comprises aluminum oxide.

9. The process of claim 1, wherein the metal oxide is selected from the group consisting of acidic aluminum oxide, basic aluminum oxide, neutral aluminum oxide, and combinations thereof.

10. The process of claim 1, wherein the metal oxide is added to the solution in an amount of from about 2% w/v to about 20% w/v.

11. The process of claim 1, wherein the metal oxide is added to the solution in an amount of from about 5% w/v to about 15% w/v.

12. The process of claim 1, further comprising subjecting the solution and metal oxide to physical agitation by a method selected from the group consisting of mixing, blending, stirring, sonication, shaking, and combinations thereof.

13. The process of claim 1, wherein isolating the polymer possessing isocyanate groups from the solution occurs by a method selected from the group consisting of filtration, solvent evaporation, and combinations thereof.

14. The process of claim 1, further comprising heating the isolated polymer to a temperature of from about 30º C to about 70º C.

15. A process comprising:
forming a solution by contacting a solvent with at least one poly(ether-ester) macromer possessing isocyanate groups of the formula:
OCN-X-HNCOO-(R-A)ₙ-ROOCNH-X-NCO (II)
wherein X is a group derived from toluene, xylene, isophorone, diphenylmethane, diphenyldimethylmethane, dibenzyl diisocyanate, oxybis(phenylisocyanate), tetramethylxylylene, hexamethylene, tetramethylene, lysine, ethylated lysine, and combinations thereof,
A is a group derived from an aliphatic diacid,
R is a group derived from a polyethylene glycol,
n is from about 1 to about 10;
adding to the solution a metal oxide selected from the group consisting of acidic aluminum oxide, basic aluminum oxide, neutral aluminum oxide, and combinations thereof, in an amount from about 2% w/v to about 20% w/v; and
isolating the at least one poly(ether-ester) macromer possessing isocyanate groups from the solution.

16. The process as in claim 15, wherein A is a group derived from adipic acid, and R is a group derived from a component selected from the group consisting of polyethylene glycol 400, polyethylene glycol 600 and polyethylene glycol 900.

17. The process as in claim 15, wherein the solvent comprises an organic solvent selected from the group consisting of tetrahydrofuran, dioxane, dimethylformamide, dichloromethane, ethyl acetate, and combinations thereof.

18. The process of claim 15, wherein the metal oxide is added to the solution in an amount of from about 5% w/v to about 15% w/v.

19. The process of claim 15, further comprising subjecting the solution and metal oxide to physical agitation by a method selected from the group consisting of mixing, blending, stirring, sonication, shaking, and combinations thereof.

20. The process of claim 15, wherein isolating the at least one poly(ether-ester) macromer possessing isocyanate groups from the solution occurs by a method selected from the group consisting of filtration, solvent evaporation, and combinations thereof.
